# EUROPEAN PATENT SPECIFICATION

(11) **EP 1 855 579 B1**
(45) Date of publication and mention of the grant of the patent: **05.10.2011**
(21) Application number: 06718676.7
(22) Date of filing: 17.01.2006
(51) Int. Cl.: A61K 8/34, A61Q 17/00

(54) **HYDROALCOHOLIC ANTIMICROBIAL COMPOSITION WITH SKIN HEALTH BENEFITS**
HYDROALKOHOLISCHE ANTIMIKROBIELLE ZUSAMMENSETZUNG MIT VORTEILEN FÜR GESUNDE HAUT
COMPOSITION ANTIMICROBIENNE AQUEUSE A BASE D'ALCOOL BENEFIQUE POUR LA PEAU

(30) Priority: 08.03.2005 US 75287
(43) Date of publication of application: 21.11.2007
(73) Proprietor: ECOLAB INC., St. Paul, MN 55102-2233 (US)
(72) Inventor: LITTAU, Cheryl, A., Apple Valley, MN 55124 (US); LE, Mai, T., Maplewood, MN 55109 (US)
(74) Representative: Polypatent
(86) International application number: PCT/US2006/001637
(87) International publication number: WO 2006/096239

(56) References cited:
- EP-A- 0 848 907
- WO-A-2005/051341
- US-A- 3 787 566
- US-A- 6 130 253
- US-A1- 2004 102 429
- US-B1- 6 217 885
- US-B1- 6 592 880
- M. SCHLOSSMAN (ED.): "The chemistry and manufacture of cosmetics: formulating. Vol.2, ed.3" 2000, ALLURED PUB. , USA 277870 , XP002390779 page 237
- H. MORTON: "The relationship of concentration and germicidal efficiency of ethyl alcohol." ANNALS OF THE NEW YORK ACADEMY OF SCIENCES, vol. 52, 1950, pages 191-196, XP008066591
- T. Donegan et al.: "Topical antimicrobial drug products for over-the-counter human use...", , 2003, Retrieved from the Internet: URL:http://www.fda.gov/ohrms/dockets/daily s/03/sept03
- M.C. Martini et al.: "Actifs et additifs en cosmétologie", 1992, Lavoisier * page 240 - page 241 * * page 269 - page 271 *

## Description

### FIELD OF THE INVENTION

The invention pertains to an alcohol based antimicrobial skin care composition. The invention also pertains to an alcohol based antimicrobial skin care composition with skin health benefits including moisturization and skin barrier maintenance effects. The invention further pertains to an alcohol based antimicrobial skincare composition that is a stable emulsion that has a certain viscosity to it that provides containment, for example on a user's hands. Finally, the invention pertains to an alcohol based antimicrobial skincare composition that may be used in the healthcare industry, for example as a surgical scrub, healthcare personnel handwash, antiseptic for injection sites, or patient pre-operative site preparation.

### BACKGROUND

Proper skin care has long been cited as an effective way of reducing the spread of germs, diseases, and other contaminants. Proper skin care is especially important in industries where bacteria are particularly problematic such as the healthcare industries, patient care industries, and the food and beverage industries.

In the healthcare and patient care industries, personnel are constantly exposing their hands to a variety of skin care products including surgical scrubs, hand washes, and waterless hand sanitizers. Over a period of time, this constant exposure to skin care products causes the skin to become dry and irritated and eventually breaks down the skin's barrier function, increasing the risk of infection to healthcare and patient care providers. Further, products that cause skin irritation or dryness will discourage healthcare and patient care providers from using such products as frequently as optimally required, which increases the risk of spreading germs and diseases, such as hospital acquired infections.

US 2004/0102429 A1 discloses a composition with 58% ethanol, more than one preservative, farnesol as a terpenoid, and more than one skin conditioner, thickener and emulsifier (paragraph [0112]).

US 3,787,566 describes in col. 8 and 9 a composition, for air sanitizing comprising isopropanol, citral as terpenoid, a cationic preservative and conditioner and glycols as well as a foam composition containing alcohol, a preservative, citral as terpenoid and skin conditioner.

Antimicrobial skin care compositions have been previously described. See U.S. Pat. Nos. 6,319,958 and 6,534,069. However, previously described skin care compositions do not provide the advantages of the present invention.

When formulating hand care products, it may be beneficial to form a composition having a certain viscosity to it so that the composition is considered thick or thickened. There are multiple ways of making a thickened composition, however, one method is to form an emulsion. An emulsion refers to a combination of two immiscible liquids (i.e. oil and water) where one liquid is dispersed, but not dissolved in the other. Forming a stable emulsion is often difficult and there are a number of things that cause emulsions to "break" or separate out into two phases. One generally accepted method of breaking emulsions is to add an alcohol.

It is against this background that the present invention has been made.

### SUMMARY

Surprisingly, it has been discovered that an antimicrobial skin care product with unexpected stability, efficacy and skin health benefits can be achieved through a synergistic combination of antimicrobial agents, preservatives, and skin conditioners. In particular, it has been discovered that the combination of an alcohol, a preservative, a thickener, an emulsifier, a terpenoid, and an additional skin conditioner, creates an emulsion with unexpected stability, unexpected antimicrobial efficacy, and unexpected skin health benefits.

The present invention therefore provides the following:
(1) An antimicrobial surgical scrub composition comprising:
   a) from 50 to 95 wt.% of a C₂-C₄ alcohol;
   b) a preservative selected from the group consisting of phenolics, halogen compounds, quaternary ammonium compounds, metal derivatives selected from the group consisting of silver borosilicate, silver magnesium aluminium phosphate, copper usnate, amines, alkanolamines, nitro-containing compounds, biguanides, anilides, organosulfur compounds, sulfur-nitrogen compounds, and mixtures thereof;
   c) a terpenoid selected from the group consisting of farnesol, nerolidol, bisabolol, apritone, and mixtures thereof;
   d) a skin conditioner selected from the group consisting of emollients, humectants, occlusive agents, and mixtures thereof;
   e) a cellulosic thickener; and
   f) an alkylene oxide ether of a fatty alcohol as emulsifier,
      wherein the composition has a viscosity of 1800 to 15000 centipoise as determined using a Brookfield RVT rotational viscometer using spindle #3 at 10rpm at 25°C.
(2) The composition of (1), wherein the alcohol is ethanol.
(3) The composition of (1), wherein
   a) the alcohol is present from 50 to 95 wt. %;
   b) the preservative is present from 0.5 to 3 wt. %;
   c) the terpenoid is present from 0.005 to 5 wt. %;
   d) the skin conditioner is present from 0.01 to 15 wt. %;
   e) the thickener is present from 0.01 to 5 wt. %; and
   f) the emulsifier is present from 0.1 to 8 wt. %.
(4) The composition of (1), wherein the composition is further comprising additional functional ingredients selected from the group consisting of skin feel improvers, antioxidants, fragrances, dyes, and mixtures thereof.
(5) The composition of (1), wherein the composition has a viscosity from 2,000 to 10,000 centipoise.
(6) The composition of (1), wherein the preservative is selected from the group consisting of polyaminopropylbiguanide, benzethonium chloride, benzalkonium chloride, and mixtures thereof.
(7) The composition of (1), wherein the composition produces at least a 2.5 log reduction of *Serratia marcescens* within 5 minutes of contact.
(8) The composition of (1), wherein the composition produces at least a 3 log reduction of *Serratia marcescens* within 5 minutes of contact.
(9) The composition of (1), wherein the composition produces at least a 3.5 log reduction of at least a 5 log baseline population of resident flora within 1 minute of contact after 5 days of use.
(10) The composition of (1), wherein the composition produces at least a 4.0 log reduction of at least a 5 log baseline population of resident flora within 1 minute of contact after five days of use.
(11) The composition of (1), wherein the composition is substantially free of anionic ingredients.

The present invention also provides:
(12) An antimicrobial surgical scrub foam composition comprising:
   a) from 50 to 95.wt.% of a C₂-C₄ alcohol;
   b) a preservative selected from the group consisting of phenolics, halogen compounds, quaternary ammonium compounds, metal derivatives selected from the group consisting of silver borosilicate, silver magnesium aluminium phosphate, copper usnate, amines, alkanolamines, nitro-containing compounds, biguanides, anilides, organosulfur compounds, sulfur-nitrogen compounds, and mixtures thereof;
   c) a terpenoid selected from the group consisting of farnesol, nerolidol, bisabolol, apritone, and mixtures thereof;
   d) a skin conditioner selected from the group consisting of emollients, humectants, occlusive agents, and mixtures thereof;
   e) a cellulosic thickener, and
   f) an alkylene oxide ether of a fatty alcohol as emulsifier.
(13) The composition of (12), wherein the composition comprises a propellant and is dispensed as a mousse.
(14) The composition of (12), wherein the composition comprises a propellant and is dispensed as an aerosol.

### DETAILED DESCRIPTION OF SOME EMBODIMENTS

### Definitions

For the following defined terms, these definitions shall be applied, unless a different definition is given in the claims or elsewhere in this specification.

Weight percent, percent by weight, % by weight, wt %, and the like are synonyms that refer to the concentration of a substance as the weight of that substance divided by the weight of the composition and multiplied by 100.

The recitation of numerical ranges by endpoints includes all numbers subsumed within that range (e.g. 1 to 5 includes 1, 1.5, 2, 2.75, 3, 3.80, 4 and 5).

As used in this specification and the appended claims, the singular forms "a," "an," and "the" include plural referents unless the content clearly dictates otherwise. Thus, for example, reference to a composition containing "a compound" includes a mixture of two or more compounds. As used in this specification and the appended claims, the term "or" is generally employed in its sense including "and/or" unless the content clearly dictates otherwise.

The use of the terms "antimicrobial" in this application does not mean that any resulting products are approved for use as an antimicrobial agent.

The terms "skin care," "skin care product," "skin care composition," and the like refer to the skin of a mammal and/or compositions or products that may be applied to the skin of a mammal. Included within these terms are compositions that are applied as hand care products, or as body products such as surgical patient site preparations.

### Alcohol Based Antimicrobial Skincare Composition

As discussed above, the invention generally relates to an alcohol based antimicrobial skin care composition (hereinafter referred to as "the composition"). In some embodiments, the composition has skin health benefits including moisturization and skin barrier maintenance effects. In some embodiments, the composition is a stable emulsion that has some viscosity to it in order to provide containment, for example on a user's hands. In some embodiments, the composition may be used in industries where skin care or hand care is especially important such as the healthcare industries, patient care industries, and food and beverage industries. In some embodiments, the composition may be used in the healthcare industry as a surgical scrub, a healthcare personnel handwash, an antiseptic for injection sites, or a patient pre-operative site preparation.

Surprisingly, it has been discovered that an antimicrobial skincare product with unexpected stability, efficacy and skin health benefits can be achieved through a synergistic combination of antimicrobial agents, preservatives, and skin conditioners. In particular, it has been discovered that the combination of an alcohol, a preservative, a thickener, an emulsifier, a terpenoid, and an additional skin conditioner, creates an emulsion with unexpected stability, unexpected antimicrobial efficacy, and unexpected skin health benefits.

The present compositions can be used in a variety of industries and especially in the healthcare and patient care industries as a surgical scrub, healthcare personnel handwash, and waterless hand sanitizer. The present compositions may be used as either a leave on product or as a rinse off product. When used as a leave on product, a user will apply the composition to the skin until the composition has either been absorbed into the skin or evaporated off. When used as a rinse off product, the user will apply the composition to the skin and then rinse, off any excess product with water. The compositions are preferably used as a leave on product in order to maximize the antimicrobial features of the composition.

It is also understood that the present invention may be used as a foaming composition and that in some embodiments, the composition may be formulated as a water-thin liquid that has increased efficacy and skin health benefits.

It is generally recognized in the industry that alcohols such as ethanol cause emulsions to break However, in the present invention, it has been observed that the presence of ethanol contributes positively to the stability and the viscosity of the emulsion.

### Alcohol

In addition to water, the composition includes an alcohol. The alcohol is a C₂-C₄ alcohol. Examples of suitable alcohols include ethanol, propanols, and butanols. The alcohol is preferably ethanol.

The composition may contain one alcohol, or a mixture of two or more alcohols. The alcohol is preferably present in the composition in an amount from about 50 to about 95 wt. %, from about 60 to about 90 wt. %, and from about 62 to about 75 wt. %.

### Preservative

The composition includes a preservative selected from phenolics, halogen compounds, quaternary ammonium compounds, metal derivatives selected from silver borosilicate, silver magnesium aluminum phosphate, copper usnate, amines, alkanolamines, nitro-containing compounds, biguanides, analides, organosulfur and sulfur-nitrogen compounds and mixtures thereof. Examples of phenolic antimicrobial agents include pentachlorophenol, orthophenylphenol, chloroxylenol, .p-chloro-m-cresol, p-chlorophenol, chlorothymol, m-cresol, o-cresol, p-cresol, isopropyl cresols, mixed cresols, phenoxyethanol, phenoxyethylparaben, phenoxyisopropanol, phenyl paraben, resorcinol, and derivatives thereof. Examples of halogen compounds include trichlorohydroxy diphenyl ether (Triclosan), sodium trichloroisocyanurate, sodium dichloroisocyanurate, iodine-poly(vinylpyrolidin-onen) complexes, and bromine compounds such as 2-bromo-2-nitropropane-1,3-diol, and derivatives thereof. Examples of quaternary ammonium compounds include benzalkonium chloride, benzethonium chloride, behentrimonium chloride, cetrimonium chloride, and derivatives thereof. Examples of amines and nitro containing compounds include hexahydro-1,3,5-tris(2-hydroxyethyl)-s-triazine, dithiocarbamates such as sodium dimethyldithiocarbamate, and derivatives thereof.

Examples of biguanides include polyaminopropyl biguanide and chlorhexidine gluconate.

In some preferred embodiments, the composition includes more than one preservative, where one preservative is selected from one class of preservatives (i.e. quaternary ammonium compound), and at least one other preservative is selected from a different class of preservatives (i.e. biguanides). The preservative is preferably benzethonium chloride, polyaminopropyl biguanide, or mixtures thereof.

The present invention includes raw materials or ingredients that perform specific functions. In some embodiments, raw materials and ingredients that perform more than one function may be selected and in some cases are preferred. For example, it may be desirable to select preservatives that act as preservatives and also are considered skin conditioners.

The preservative is preferably present in the composition in an amount from about 0 to about 3 wt. %, from about 0.1 to about 2 wt. %, and from about 0.2 to about 1 wt. %.

### Thickener

The composition includes a cellulosic thickener so that the composition is a viscous liquid, gel, or semisolid that can be easily applied to and rubbed on the skin. The thickener may thicken the composition by either thickening the aqueous portions of the composition, or by thickening the non-aqueous portions of the composition. Some examples of cellulosic thickeners include carboxymethyl hydroxyethylcellulose, cellulose, hydroxybutyl methylcellulose, hydroxyethylcellulose, hydroxypropylcellulose, hydroxypropyl methyl cellulose, methylcellulose, microcrystalline cellulose, sodium cellulose sulfate, and the like.

The composition may contain one thickener or a mixture of two or more thickeners. Preferred thickeners do not adversely react with other raw materials in the composition. For example, in some embodiments, the composition may include cationic raw materials such as quaternary ammonium preservatives. In those embodiments, it may be preferred to have a nonionic or cationic thickener that does not adversely react with the cationic raw materials. It is understood that a person skilled in the art will know how to select an appropriate thickener and control any adverse reactions through formulating. The preferred thickeners for the compositions of the invention are cellulosic ethers and quaternized cellulosic ethers such as Polyquaternium-10, commercially available as Celquat SC-230M from National Starch (Bridgewater, NJ).

The present invention includes raw materials or ingredients that perform specific functions. In some embodiments, raw materials and ingredients that perform more than one function may be selected and in some cases are preferred.

The amount of thickener present in the composition depends on the desired viscosity of the composition. The composition preferably has a viscosity from about 800 to about 20,000 centipoise, from about 1,800 to about 15,000 centipoise, and from about 2,000 to about 10,000 centipoise as determined using a Brookfield RVT rotational viscometer using spindle # 3 @ 10 rpm @ 25 °C. Accordingly, to achieve the preferred viscosities, the thickener may be present in the use composition in an amount from about 0.01 wt. % to about 5 wt. % of the total composition, from about 0.05 wt. % to about 2.5 wt. %, and from about 0.1 wt. % to about 1.5 wt. % of the total composition.

### Emulsifier

As previously discussed, the composition is preferably an emulsion which is a combination of two immiscible compositions. The composition includes at least one emulsifier in order to help stabilize the emulsion selected from the condensation products of alkylene oxides with fatty acids (i.e. alkylene oxide esters of fatty acids). These materials have the general formula RCO(X)ₙOH wherein R is a C₁₀₋₃₀ alkyl group, X is --OCH₂CH₂-- (i. e. derived from ethylene glycol or oxide) or -OCH₂CHCH ₃-- (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula RCO(X)ₙOOCR wherein R is a C₁₀₋₃₀ alkyl group, X is OCH₂CH₂-(i.e. derived from ethylene glycol or oxide) or --OCH₂CHCH₃-(i.e. derived from propylene glycol or oxide), and n is an integer from about 6 to about 100. Additional nonionic surfactants include condensation products of alkylene oxides with fatty alcohols (alkylene oxide ethers of fatty alcohols) where R is a C₈-C₃₀ alkyl group, X is OCH₂CH₂-- and n is an integer from about 1 to about 200.

The hydrophilic surfactants useful herein can additionally include any of a wide variety of cationic, anionic, zwitterionic, and amphoteric surfactants such as are known in the art. See, e.g., McCutcheon's, Detergents and Emulsifiers, North American Edition (1986), published by Allured Publishing Corporation; U.S. Pat. No. 5,011,681 to Ciotti et al., issued Apr. 30, 1991; U.S. Pat. No. 4,421,769 to Dixon et al., issued Dec. 20, 1983; and U.S. Pat. No. 3,755, 560 to Dickert et al., issued Aug. 28, 1973.

In addition, a wide variety of anionic surfactants are also useful herein. See, e.g., U.S. Pat. No. 3,929,678, to Laughlin et al., issued Dec. 30, 1975. Exemplary anionic surfactants include the alkoyl isethionates (e.g., C₁₂-C₃₀), alkyl and alkyl ether sulfates and salts thereof, alkyl and alkyl ether phosphates and salts thereof, alkyl methyl taurates (e.g., C₁₂-C₃₀), and soaps (e.g., alkali metal salts, e.g., sodium or potassium salts) of fatty acids.

Amphoteric and zwitterionic surfactants in addition are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C₈-C₁₈) and one contains an anionic water solubilising group, e.g., carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants ate those selected from the group consisting of betaines, sultaines, hydroxysultaines, and branched and unbranched alkanoyl sarcosinates, amine oxides, and mixtures thereof.

The composition may contain one emulsifier or a mixture of two or more emulsifiers. Preferred emulsifiers do not adversely react with other raw materials in the composition. For example, in some embodiments, the composition may include cationic raw materials such as quaternary ammonium preservatives. In those embodiments, it may be preferred to have a nonionic emulsifier that does not adversely react with the cationic raw materials. It is understood that a person skilled in the art will know how to select an appropriate emulsifier and control any adverse reactions through formulating. The preferred emulsifiers for the compositions of the invention are alkylene oxide ethers of fatty alcohols such as Laureth-3 and Laureth-23, commercially available as Genapol LA-030 and Genapol LA-230 from Clariant Corporation (Charlotte, NC).

The present invention includes raw materials or ingredients that perform specific functions. In some embodiments, raw materials and ingredients that perform more than one function may be selected and in some cases are preferred.

The emulsifier may be present in the composition in an amount from about 0.1 to about 8 wt. % of the total composition, from about 0.25 to about 6 wt. %, and from about 0.5 to about 4 wt. % of the total composition.

### Terpenoids

The composition includes at least one terpenoid. Terpenoids are defined as materials with molecular structures containing carbon backbones made up of isoprene (2-methylbuta-1,3-diene) units. Isoprene contains five carbon atoms and therefore, the number of carbon atoms in any terpenoid is a multiple of five. It is believed that terpenoids assist in promoting the uptake of antimicrobial compounds and preservatives by cells of bacteria and fungi, thereby increasing the efficacy of the antimicrobial compound or preservative. See U.S. Pat. No. 6,319,958 and DE 195 23 320. Examples of terpenoids include α-terpinene, cineole, citral, citronellal, citronellol, farnesol, geraniol, limonene, linalool, methone, nerolidol, terpineol, camphene, menthone, myrcene, nerol, tetrayhydrogeraniol, tetrahydrolinalool, apritone, and bisabolol. The terpenoid is preferably farnesol, nerolidol, bisabolol, or apritone.

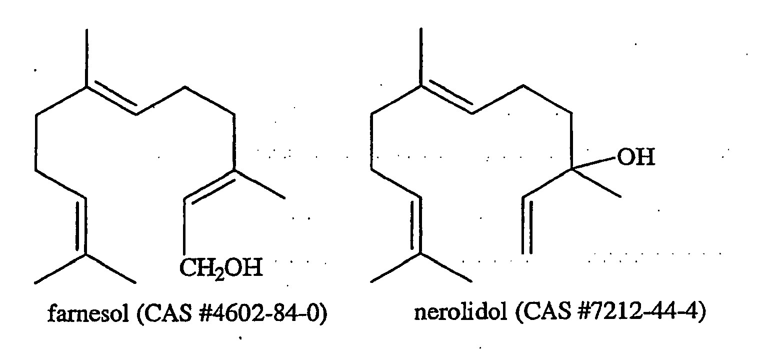

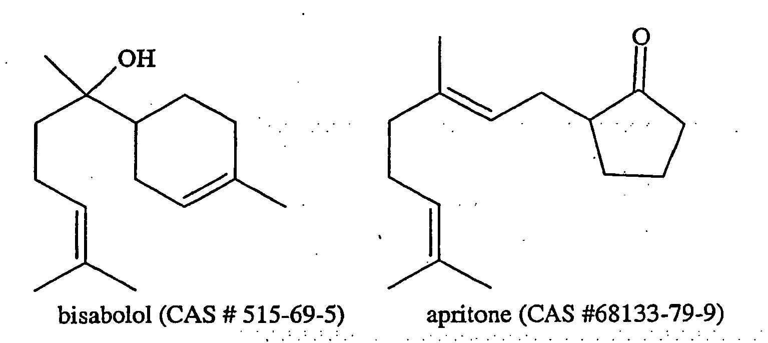

The present invention includes raw materials or ingredients that perform specific functions. In some embodiments, raw materials and ingredients that perform more than one function may be selected and in some cases are preferred. For example, it is recognized that farnesol, nerolidol, bisabolol, and apritone perform dual functions of increasing the efficacy.of the antimicrobial components and preservatives while providing skin health benefits such as anti-irritancy and skin repair.

The terpenoid is preferably present in the composition in an amount from about 0.005 to about 5 wt. %, from about 0.05 to about 2.5 wt. %, and from about 0.1 to about 1.5 wt. %.

### Skin Conditioner

The composition includes at least one skin conditioner such as an emollient, humectant, occlusive agent, or other moisturizer to provide moisturizing, skin softening, skin barrier maintenance, anti-irritation, or other skin health benefits. Some non-limiting examples of emollients include stearoxytrimethylsilane, alkyl benzoate, silicone oils, dimethicone, myristyl myristate, cetyl myristate, glyceryl dioleate, methyl laurate, PPG-9 laurate, octyl palmitate, lanolin, propylene glycol, glycerine, fatty acids, natural oils such as almond, mineral, canola, sesame, soybean, wheat germ, corn, peanut, and olive, isopropyl myristate, myristyl alcohol, aloe vera, hydrolyzed silk protein, Vitamin E, stearyl alcohol, isopropyl palmitate, sorbitol, amino acid complexes, and polyethylene glycol. Some non-limiting examples of humectants include hydroxyethyl urea, agarose, arginine PCA, fructose, glucose, glutamic acid, glycerine, honey, lactose, maltose, propylene glycol, polyethylene glycol, sorbitol and mixtures thereof. Some non-limiting examples of occlusive agents include petrolatum, shea butter, alkyl dimethicones, avocado oil, balm mint oil, canola oil, cod liver oil, corn oil, methicone, mineral oil, olive oil, phenyl trimethicone, trimyristin, soybean oil, stearyl stearate, synthetic wax, or mixtures thereof. Some non-limiting examples of other moisturizers include cholesterol, cystine, hyaluronic acid, keratin, lecithin, egg yolk, glycine, PPG-12, panthenol, retinol, salicylic acid, vegetable oil, and mixtures thereof. Finally, some non-limiting examples of anti-irritants include bisabolol and panthenol.

The composition may include one skin conditioner or a mixture of more than one skin conditioner. In some preferred embodiments, the skin conditioner is a mixture of at least one emollient, and at least one humectant. In some preferred embodiments, the skin conditioner is aloe vera, hydroxyethyl urea, polyethylene glycol, panthenol, hyaluronic acid, alkyl benzoate, stearoxytrimethylsilane, myristyl myristate, and mixtures thereof. It is recognized that some of these preferred skin conditioners have a dual function. For example, myristyl myristate and stearoxytrimethylsilane also act as thickeners.

The present invention includes raw materials or ingredients that perform specific functions. In some embodiments, raw materials and ingredients that perform more than one function may be selected and in some cases are preferred.

A person skilled in the art will recognize the different strengths of different skin conditioners and formulate accordingly. In some embodiments, the skin conditioner is preferably present in the composition in an amount from about 0.01 to about 20 wt. %, from about 0.1 to about 15 wt. %, and from about 1 to about 10 wt. %.

### Additional Functional Ingredients

Additional functional ingredients may be used to improve the effectiveness of the composition. Some non-limiting examples of such additional functional ingredients include skin feel improvers, foaming agents, antioxidants, fragrances, dyes, and mixtures thereof. The present invention includes raw materials or ingredients that perform specific functions. In some embodiments, raw materials and ingredients that perform more than one function may be selected and in some cases are preferred.

### Skin Feel Improver

The composition may optionally include a skin feel improver for enhancing the "feel" of the composition on a user's skin or hands. For example, it may be undesirable for a composition to have a scaly or gritty texture when applied to a user's skin or after the multiple applications of the composition. Some non-limiting examples of skin feel improvers include silicone polymers and copolymers such as amodimethicone, cyclomethicone, Bis-PEG/PPG-20/20 dimethicone, and stearoxytrimethylsilane, naturally occurring or synthetic fatty acid esters or ethers, and polyalkylene glycols.

If a skin feel improver is included, it is preferably present in the composition in an amount from about 0.001 to about 5 wt. %, from about 0.01 to about 3 wt. %, and from about 0.1 to about 2 wt. %.

### Foaming Agents

It may be desirable to dispense the present compositions as an aerosol foam or mousse. If dispensing as an aerosol foam or mousse, a propellant may be included. Some non-limiting examples of propellants include chlorofluorocarbons (CFC's), hydrochlorofluorocarbons (HCFC's), hydrofluorocarbons (HFCs), prefluorinated alkanes (C₁-C₅), nitrous oxide, dimethyl ether, and the like. Examples of suitable dispensers include the QuikCare Dispensers, commercially available from Ecolab Inc., or the aerosol cans commercially available from Exal.

### Antioxidant

The composition may optionally include an antioxidant for improved skin condition through the removal of free radicals, and improved product stability. Some non-limiting examples of antioxidants include ascorbic acid and ascorbic acid derivatives, BHA, BHT, betacarotene, cysteine, erythorbic acid, hydroquinone, tocopherol and tocopherol derivatives, and the like.

If an antioxidant is included, it is preferably present in the composition in an amount from about 0.001 to about 2 wt. %, from about 0.01 to about 1 wt. %, and from about 0.05 to about 0.5 wt. %.

### Fragrance

The composition may optionally include a fragrance. Examples of possible fragrances include natural oils or naturally derived materials, and synthetic fragrances such as hydrocarbons, alcohols, aldehydes, ketones, esters, lactones, ethers, nitriles, and polyfunctionals. Non-limiting examples of natural oils include the following: basil (*Ocimum basilicum*) oil, bay (*Pimento acris*) oil, bee balm (*Monarda didyma*) oil, bergamot (*Citrus aurantium bergamia*) oil, cardamom (*Elettaria cardamonmum*) oil, cedarwood (*Cedrus atlantica*) oil, chamomile (*Anthemis nobilis*) oil, cinnamon (*Cinnamomum cassia*) oil, citronella (*Cymbopogon nardus*) oil, clary (*Salvia sclarea*) oil, clove (*Eugenia caryophyllus*) oil, cloveleaf (*Eufenia caryophyllus*) oil, *Cyperus esculentus* oil, cypress (*Cupressus sempervirens*) oil, *Eucalyptus citriodora* oil, geranium maculatum oil, ginger (*Zingiber officinale*) oil, grapefruit (*Citrus grandis*) oil, hazel (*Corylus avellana*) nut oil, jasmine (*Jasminum officinale*) oil, *Juniperus communis* oil, *Juniperus oxycedrus* tar, *Juniperus virginiana* oil, kiwi (*Actinidia chinensis*) water, lavandin (*Lavandula hybrida*) oil, lavender (*Lavandula angustifolia*) oil, lavender (*Lavandula angustifolia*) water, lemon (*Citrus medica limonum*) oil, lemongrass (*Cymbopogon schoenanthus*) oil, lime (*Citrus aurantifolia*) oil, linden (*Tilia cordata*) oil, linden (*Tilia cordata*) water, mandarin orange (*Citrus nobilis*) oil, nutmeg (*Myristica fragrans*) oil, orange (*Citrus aurantium dulcis*) flower oil, orange (*Citrus aurantium dulcis*) oil, orange (*Citrus aurantium dulcis*) water, patchouli (*Pogostemon cablin*) oil, peppermint (*Menthe piperita*) oil, peppermint (*Menthe peperita*) water, rosemary (*Rosmarinus officinalis*) oil, rose oil, rose (*Rosa damascena*) extract, rose (*Rosa multiflora*) extract, rosewood (*Aniba rosaeodora*) extract, sage (*Salvia officinalis*) oil, sandalwood (*Santalum album*) oil, spearmint (*Menthe viridis*) oil, tea tree (*Melaleuca altenzifolia*) oil, and ylang ylang (*Cananga odorata*) oil. Some non-limiting examples of synthetic hydrocarbon fragrances include caryophyllene, β-farnesene, limonene, α-pinene, and β-pinene. Some non-limiting examples of synthetic alcohol fragrances include Bacdanol, citronellol, linalool, phenethyl alcohol, and α-terpineol (R=H). Some non-limiting examples of synthetic aldehyde fragrances include 2-methyl undecanal, citral, hexyl cinnamic aldehyde, isocycolcitral, lilial, and 10-undecenal. Some non-limiting examples of synthetic ketone fragrances include cashmeran, α-ionone, isocyclemone E, koavone, muscone, and tonalide. Some non-limiting examples of synethetic ester fragrances include benzyl acetate, 4-*t-*butylcyclohexyl acetate (cis and trans), cedryl acetate, cyclacet, isobornyl acetate, and α-terpinyl acetate (R=acetyl). Some non-limiting examples of synthetic lactone fragrances include coumarin, jasmine lactone, muskalactone, and peach aldehyde. Some non-limiting examples of synthetic ether fragrances include Ambroxan, Anther, and Galaxolide. Some non-limiting examples of synthetic nitrile fragrances include cinnamonitrile and gemonitrile. Finally, some non-limiting examples of synthetic polyfunctional fragrances include amyl salicylate, isoeugenol, Hedione, heliotropine, Lyral, and vanillin.

The composition may include a mixture of fragrances including a mixture of natural and synthetic fragrances. The fragrance can be present in a composition in an amount up to about 5 wt. %, preferably from about 0.01 to about 3 wt. %, from about 0.05 to about 1 wt. %, and from about 0.1 to about 0.2 wt. %.

### Dye

The composition may optionally include a dye. Examples of dyes include any water soluble or product soluble dye, any FD&C or D&C approved dye, Blue 1, FD&C Yellow 5, Resorcin Brown, Red 40, Direct Blue 86 (Miles), Basic Violet 10 (Clariant), Acid Yellow 23 (GAF), Acid Yellow 17 (Sigma Chemical), Sap Green (Keyston Analine and Chemical), Metanil Yellow (Keyston Analine and Chemical), Acid Blue 9 (Hilton Davis), Sandolan Blue/Acid Blue 182 (Clariant), Hisol Fast Red (Capitol Color and Chemical), Fluorescein (Capitol Color and Chemical), Acid Green 25 (Ciba Specialties), and the like. The dye is preferably a water soluble dye. Also, the dye is preferably a FD&C or D&C approved dye.

The dye can be present in a use composition in an amount up to about 0.5 wt. %, preferably from about 0.00001 to about 0.1 wt. %, from about 0.0001 to about 0.01 wt. %, and from about 0.0001 to about 0.0005 wt. %.

The present compositions can be dispensed from a variety of dispensers including traditional push bar handcare dispensers and the foaming dispensers previously discussed.

### Methods of Preparation

The composition may be prepared in variety of ways, however care needs to be taken to make sure that the resulting composition forms a stable emulsion. The formation of a stable emulsion can depend on the order the raw materials are added together and the temperature. In a preferred embodiment, the composition is prepared by first forming a water phase by combining water, and water soluble ingredients, and stirring until the solution is clear. Next, an oil phase is prepared by combining the water insoluble ingredients. It may be necessary to heat the oil phase in order to melt the ingredients. Once the oil phase and water phase are prepared, they are preferably heated to the same temperature and then combined with stirring. It was found especially effective to add the cellulosic thickener immediately after the water phase and oil phase were combined but before cooling the composition down. Once the composition is cooled below 50°C (90°F), the ethanol may be added in portionwise until the addition of the ethanol is complete. After the addition of the ethanol and the composition has cooled, the terpenoids may be added along with the fragrance.

For a more complete understanding of the invention, the following examples are given to illustrate some embodiment. These examples and experiments are to be understood as illustrative. All parts are by weight, except where it is contrarily indicated.

### EXAMPLES

The following chart provides a brief explanation of certain chemical components used in the following examples:

**Table 1 Trade Names and Corresponding Descriptions of Some Chemicals Used in the Examples**

| **Trademark** | **INCI Name** | **Description** | **Provider** |
|---|---|---|---|
| Ethanol | SDA 40-B | Active Antimicrobial | |
| Crodamol MM | Myristyl Myristate | Skin Conditioner | Croda Corp. |
| Hydrovance | Hydroxyethyl Urea | Skin Conditioner | National Starch |
| Genapol LA-230 | Laureth-23 | Emulsifier | Clariant Corp. |
| Panthenol 50W | Panthenol | Skin Conditioner | BASF |
| Carbowax 1450 | Polyethylene Glycol | Skin Conditioner | Dow Chemical Co. |
| | 1450 | | |
| Celquat SC- | Polyquaternium-10 | Thickener | National Starch |
| 230M | | | |
| Genapol LA-030 | Laureth-3 | Emulsifier | Clariant Corp. |
| SilCare Silicone | Stearoxy | Skin Conditioner | Clariant Corp. |
| 1M71 | trimethylsilane | | |
| Abil B-8832 | Bis-PEG/PPG-20/20 | Skin Feel Improver | Degussa |
| | Dimethicone | | |
| Cosmocil CQ | Polyamino | Preservative | Arch Chemical Co. |
| | propylbi guanide | | |
| Crodamol AB | C12-C15 Alkyl | Skin Conditioner | Croda |
| | Benzoate | | |
| Farnesol | Farnesol | Terpenoid | Symrise |
| Arlasilk | Cocamidopropyl | Skin Conditioner | Uniqema |
| Phospholipid | PG-dimonium | | |
| PTC | chloride phosphate | | |
| Cetrimonium | | Skin Conditioner | Clariant Corp. |
| Chloride | | | |
| Dragosantol | Bisabolol | Terpenoid | Symrise |
| Lonzaguard | Benzethonium | Preservative | Lonza Corp. |
| | Chloride | | |
| Vitamin E | Tocopheryl Acetate | Skin | Roche |
| Acetate | | Conditioner/Antioxidant | |
| | | Fragrance | |
| Ritaloe 200 | Aloe Barbadensis | Skin Conditioner | Rita Corp. |
| | Leaf Juice | | |
| Biocare-Polymer | Hyaluronic Acid | Skin Conditioner | Dow Chemical |
| BHA-10 | | | Cop. |

### Example 1

Example 1 compares the viscosities of several compositions of the invention. The hydroalcoholic antimicrobial compositions of the invention were prepared as follows: Ingredients 1-8 in Table 2 were added to the main mix vessel, stirred well to insure thorough mixing and heated to approximately 77.7°C (40°F). Ingredients 9-15 in Table 2 were combined in a separate mixing vessel and heated to approximately 83.3°C (150°F) with mixing. The mixture of ingredients 9-15 were added all at once to the main mix vessel with good agitation, to form an emulsion. Ingredient 16 was added to the mixture and the mixture was held at 77.7°C (140°F) for approximately 30 minutes. After 30 minutes of mixing at temperature, the mixture was cooled to <50°C (90°F), over a period of >30 minutes. Ingredient 21 was added to the thickened emulsion, followed by ingredients 17-20. Table 2 describes the formulas and their viscosities.

**Table 2**

| | **Ingredient** | **Formula** | **Formula** | **Formula** | **Formula** | **Formula** | **Formula** |
|---|---|---|---|---|---|---|---|
| | | **A** | **B** | **C** | **D** | **E** | **F** |
| 1 | Carbowax | 0.5 | 0.5 | 0.5 | 0.5 | 0.5 | 0.5 |
| | 1450 | | | | | | |
| 2 | Hydrovance | 2.5 | 2.5 | 2.5 | 2.5 | 2.5 | 2.5 |
| 3 | Ritaloe 200 | 0.0025 | 0.0025 | 0.0025 | 0.0025 | 0.0025 | 0.0025 |
| 4 | Lonzagard | 0.08 | 0.08 | 0.12 | 0.12 | 0.12 | 0.12 |
| 5 | Panthenol | 1.0 | 1.0 | 1.0 | 1.0 | 1.0 | 1.0 |
| | 50W | | | | | | |
| 6 | Cosmocil CQ | 1.5 | 1.5 | 1.5 | 1.5 | 1.5 | 1.5 |
| 7 | Biocare- | 0.1 | 0.1 | 0.1 | 0.1 | 0.1 | 0.1 |
| | Polymer | | | | | | |
| | BHA-10 | | | | | | |
| 8 | Cetrimonium | 0.12 | 0.06 | 0.0 | 0.0 | 0.0 | 0.0 |
| | Chloride | | | | | | |
| 9 | SilCare | 0.25 | 0.25 | 0.25 | 0.25 | 0.25 | 0.25 |
| | Silicone | | | | | | |
| | 1M71 | | | | | | |
| 10 | Genapol LA- | 0.80 | 0.80 | 0.80 | 0.80 | 0.80 | 0.80 |
| | 230 | | | | | | |
| 11 | Crodamol | 4.0 | 4.0 | 4.0 | 4.0 | 4.0 | 4.0 |
| | MM | | | | | | |
| 12 | Genapol LA- | 0.40 | 0.40 | 0.40 | 0.40 | 0.40 | 0.40 |
| | 030 | | | | | | |
| 13 | Arlasilk | 0.2 | 0.2 | 0.2 | 0.2 | 0.2 | 0.2 |
| | Phospholipid | | | | | | |
| | PTC | | | | | | |
| 14 | Abil B-8832 | 0.4 | 0.4 | 0.4 | 0.4 | 0.8 | 1.2 |
| 15 | Crodamol | 0.25 | 0.25 | 0.25 | 0.25 | 0.25 | 0.25 |
| | AB | | | | | | |
| | | | | | | | |
| 16 | Celquat SC- | 0.6 | 0.5 | 0.56 | 0.525 | 0.56 | 0.56 |
| | 230M | | | | | | |
| 17 | Farnesol | 0.20 | 0.20 | 0.20 | 0.20 | 0.20 | 0.20 |
| 18 | Dragosantol | 0.15 | 0.15 | 0.15 | 0.15 | 0.15 | 0.15 |
| 19 | Vitamin E | 0.05 | 0.05 | 0.05 | 0.05 | 0.05 | 0.05 |
| | Acetate | | | | | | |
| 20 | Fragrance | 0.005 | 0.005 | 0.005 | 0.005 | 0.005 | 0.005 |
| 21 | Ethanol | 73.00 | 73.00 | 73.00 | 73.00 | 66.00 | 58.00 |
| | Viscosity* | 6100* | 2500** | 4800* | 3800* | 500* | 70* |
| | (cps) | | | | | | |

| | | | | | | | |
|---|---|---|---|---|---|---|---|
| * Viscometer: Brookfield RVT, spindle #3, 10 rpm, 25°C **Viscometer: Brookfield RVT, spindle #2, 10 rpm, 25°C | | | | | | | |

### Example 2

Example 2 tested the ability of the compositions of the invention to reduce resident bacteria. For this example, Formula C in Table 2 was tested as specified in the US Food and Drug Administration (USFDA) Tentative Final Monograph (TFM) (FR 59:116, 17 June 19494) using a modification of ASTM E-1115-91 (The Annual Book of ASTM Standards, Vol. 11.05, pp.447-450, 1996). Here, twelve participants were instructed to apply 2 milliliters of Formula C in the palm of one hand, dip the finger tips of the opposite hand into the formula and work formula under the nails, and spread the remaining formula over all surfaces of the treated hand and forearm, up to the elbow, paying special attention to the interdigital spaces. Using another 2 milliliters of the formula, the process was repeated for the other hand and forearm. Finally, another 2 milliliters of the formula was applied to either hand and reapplied to all aspects of both hands up to the wrist. The formula was allowed to dry before the gloves were put on. The critical performance properties for the test products are: an immediate one log10 reduction in resident microorganisms on Day 1 of the test; an immediate 2 log10 reduction in resident microorganisms on Day 2 of the test; and an immediate 3 log 10 reduction in microorganisms on Day 5 of the test. Microbial counts taken approximately 6 hours after the treatment/scrub must not exceed the baseline counts.

The study was conducted to evaluate the antimicrobial effectiveness of Formula C compared to a standard product (Hibiclens, 4% chlorhexidiene gluconate, Regent Medical, Norcross, GA), utilizing a traditional brush scrubbing technique. For the control, the users were instructed to apply the Hibiclens control according to the Hibiclens use directions. Specifically, the participants wet their hands and forearms with water. Next, the users scrubbed for 3 minutes with a wet brush and about 5 milliliters of the Hibiclens control product, paying particular attention to the nails, cuticles, and interdigital spaces. A separate nail cleaner was used. Then the users rinsed thoroughly, and washed for an additional 3 minutes with 5 milliliters of Hibiclens followed by a rinse under running water. Table 3 below contains the log reductions for Formula C and the control compared to the FDA requirement. The results are the mean of the twelve participants.

**Table 3**

| Formula | Day 1, | Day 1, | Day 2, | Day 2, | Day 5, | Day 5, |
|---|---|---|---|---|---|---|
| | Immediate | 6 hour | Immediate | 6 hour | Immediate | 6 hour |
| C | 3.4 | 2.4 | 3.6 | 3.2 | 4.1 | 4.1 |
| Hibiclens | 1.6 | 1.1 | 2.3 | 2.0 | 3.9 | 3.2 |
| Control | | | | | | |
| FDA | 1 | >0 | 2 | >0 | 3 | >0 |
| requirement | | | | | | |

Formula C shows significantly better log reduction values over the entire course of the study. This demonstrates that Formula C has superior immediate reduction, upon first use, as well as superior persistent and cumulative action, as demonstrated by its increasing efficacy over the 5 day test, and by its continued high log reduction values even at the 6 hour evaluation point.

### Example 3

Example 3 tested the ability of the compositions of the invention to reduce transient microbial flora (contaminants) on the hands, using a marker organism, to evaluate it's efficacy as a Healthcare Personnel Handwash (HCPHW). Formula C in Table 2 was tested in accordance with the USFDA's tentative final monograph (Federal Register, Vol. 59, pp 31402-31452, June 17, 1994), using a modification of the procedure described in ASTM E-1174-94 (The Annual Book of Standards, Vol. 11.05, pp. 480-482, 1996). Here, sixteen participants were used for testing Formula C and four participants were used for the control. The participants were instructed to place 2 milliliters of Formula C into the palm of one hand and spread evenly over all aspects of the hand and wrist, paying particular attention to the space under the nails, cuticles, and interdigital spaces. The participants were instructed to rub their hands vigorously until dry. The critical performance properties for the test products are: a 2 log₁₀ reduction in the concentration of the marker organism (*Serratia marcescens*) within 5 minutes following first wash (application of product); and a 3 log₁₀ reduction in the concentration of the marker organism (*Serratia marcescens*) within 5 minutes following 10th wash (application of product).

The study was conducted to evaluate the antimicrobial effectiveness of Formula C compared to a standard product Hibiclens, utilizing a traditional handwashing technique. The four control participants were instructed to wet their hands with water, dispense about 5 milliliters of Hibiclens into cupped hands and wash in a vigorous manner for 15 seconds. The users were then instructed to rinse and dry their hands thoroughly. Table 4 below contains the mean log reductions and Hibiclens for Formula C compared to the FDA requirements.

**Table 4**

| Formula | Wash 1 | Wash 3 | Wash 7 | Wash 10 |
|---|---|---|---|---|
| C | 3.4 | 4.5 | 5.4 | 5.9 |
| Hibiclens Control | 3.0 | 4.0 | 4.9 | 4.3 |
| FDA requirement | 2 | -- | -- | 3 |

The results of the HCPHW study demonstrate that Formula C has efficacy well exceeding that required by the USFDA TFM. The efficacy of Formula C was also consistently greater than the Hibiclens control, using a traditional soap and water wash technique.

### Example 4

Example 4 tested the degree of moisturization of the compositions of the invention. Formula C of Table 2 was tested on human volunteers with mild to moderately dry skin, over a period of 5 days, with 4 treatments per day on the first 4 days, and 2 treatments per day on the final day, for a total of 18 treatments. The change in degree of moisturization in comparison to the baseline values was measured using two different instrumental techniques, one measuring the conductivity on the skin's surface (Skicon-200 (I.B.S. Japan) using a MT8C Probe (Measurement Technologies, Cincinnati, OH)), and one measuring the capacitance of the skin (Corneometer 820 (Courage & Khazaka, Germany)). Conductivity and capacitance measure the amount of water in the skin. More specifically, the Corneometer estimates the water content of the epidermis to an approximate depth ranging between 60-100 micrometer. The Skicon estimates the water content in the upper most layers of stratum corneum. The numbers provided are the mean change in relative skin moisturization/water content. The higher the number, the better a product is at moisturizing. The health/integrity of the skin's barrier function was also measured by comparing the trans-epidermal water loss after treatment with that prior to treatment (ServoMed EP-1 or EP-2 Evaporimeter (ServoMed AB, Sweden)). Trans-epidermal water loss measures the skin's ability to hold moisture in. The numbers provided are the change in water loss from the baseline, therefore the lower the number, the better the product is at helping the skin retain moisture. The results of this study are summarized in the table below.

**Table 5**

| Formula | Δ Moisturization | Δ Moisturization | Δ TEWL from |
|---|---|---|---|
| | from Baseline | from Baseline | Baseline |
| | (Comeometer) | (Skicon) | (ServoMed |
| | | | Evaporimeter) |
| C | 6.4 | 29.6 | 0.4 |
| Market Product I¹ | 1.8 | 4.3 | 0.2 |
| Market Product II² | 1.4 | 10.1 | 0.8 |
| Market Product III³ | -0.3 | 16.6 | 1.9 |
| Hand Lotion | 1.3 | 14.4 | 0.4 |
| Control⁴ | | | |

| | | | |
|---|---|---|---|
| 1. AVAGARD™ Surgical and Healthcare Personnel Hand Antiseptic with Moisturizers, commercially available from 3M (contains 61wet% ethanol, 1% chlorhexidine gluconate). 2. TRISEPTIN Waterless Surgical Scrub, commercially available from Healthpoint (contains 61 wt% ethanol, Zinc Pyrithione). 3. ALCARE OR Foamed Antiseptic Handrub, commercially available from Steris (contains 62 vol % ethanol). 4. Accent Plus Amino Lotion, commercially available from Ecolab Inc. (alcohol-free hand lotion). | | | |

The results summarized in Table 5 indicate that Formula C has a highly superior moisturizing effect on the skin, both in the superficial and deeper layers of the skin. In contrast, the comparative market products showed more moderate moisturization in the superficial skin layers, and in some cases actually reduced the moisture in the deeper layers of the skin. Similarly, Formula C showed no significant increase in the rate of TEWL (no different to the lotion control), while two of the three comparative products did show a significant increase, indicating that these products are disrupting the structure of the skin's barrier to evaporative water loss.

### Example 5

One example of a foam composition would use Formula B of Table 2, prepared as discussed in Example 1. The formula is filled into appropriate containers and charged with a suitable amount of propellant (i.e. between 6% and 15%). Suitable propellants include Propellant A-31 (isobutane), Propellant A-46 (propane/isobutane), Hydroflurocarbon 152A, or mixtures thereof.

The foregoing summary, detailed description, and examples provide a sound basis for understanding the invention, and some specific example embodiments of the invention. The invention resides in the claims.

## Claims

1. An antimicrobial surgical scrub composition comprising:
a) from 50 to 95 wt.% of a C₂-C₄ alcohol;
b) a preservative selected from the group consisting of phenolics, halogen compounds, quaternary ammonium compounds, metal derivatives selected from the group consisting of silver borosilicate, silver magnesium aluminium phosphate, copper usnate, amines, alkanolamines, nitro-containing compounds, biguanides, anilides, organosulfur compounds, sulfur-nitrogen compounds, and mixtures thereof;
c) a terpenoid selected from the group consisting of farnesol, nerolidol, bisabolol, apritone, and mixtures thereof;
d) a skin conditioner selected from the group consisting of emollients, humectants, occlusive agents, and mixtures thereof;
e) a cellulosic thickener; and
f) an alkylene oxide ether of a fatty alcohol as emulsifier,
wherein the composition has a viscosity of 1800 to 15000 centipoise as determined using a Brookfield RVT rotational viscometer using spindle #3 at 10rpm at 25°C.

2. The composition of claim 1, wherein the alcohol is ethanol.

3. The composition of claim 1, wherein
a) the alcohol is present from 50 to 95 wt. %;
b) the preservative is present from 0.5 to 3 wt. %;
c) the terpenoid is present from 0.005 to 5 wt. %;
d) the skin conditioner is present from 0.01 to 15 wt. %;
e) the thickener is present from 0.01 to 5 wt. %; and
f) the emulsifier is present from 0.1 to 8 wt. %.

4. The composition of claim 1, wherein the composition is further comprising additional functional ingredients selected from the group consisting of skin feel improvers, antioxidants, fragrances, dyes, and mixtures thereof.

5. The composition of claim 1, wherein the composition has a viscosity from 2,000 to 10,000 centipoise.

6. The composition of claim 1, wherein the preservative is selected from the group consisting of polyaminopropylbiguanide, benzethonium chloride, benzalkonium chloride, and mixtures thereof.

7. The composition of claim 1, wherein the composition produces at least a 2.5 log reduction of *Serratia marcescens* within 5 minutes of contact.

8. The composition of claim 1, wherein the composition produces at least a 3 log reduction of *Serratia marcescens* within 5 minutes of contact.

9. The composition of claim 1, wherein the composition produces at least a 3.5 log reduction of at least a 5 log baseline population of resident flora within 1 minute of contact after 5 days of use.

10. The composition of claim 1, wherein the composition produces at least a 4.0 log reduction of at least a 5 log baseline population of resident flora within 1 minute of contact after five days of use.

11. The composition of claim 1, wherein the composition is substantially free of anionic ingredients.

12. An antimicrobial surgical scrub foam composition comprising:
a) from 50 to 95 wt.% of a C₂-C₄ alcohol;
b) a preservative selected from the group consisting of phenolics, halogen compounds, quaternary ammonium compounds, metal derivatives selected from the group consisting of silver borosilicate, silver magnesium aluminium phosphate, copper usnate, amines, alkanolamines, nitro-containing compounds, biguanides, anilides, organosulfur compounds, sulfur-nitrogen compounds, and mixtures thereof;
c) a terpenoid selected from the group consisting of farnesol, nerolidol, bisabolol, apritone, and mixtures thereof;
d) a skin conditioner selected from the group consisting of emollients, humectants, occlusive agents, and mixtures thereof;
e) a cellulosic thickener, and
f) an alkylene oxide ether of a fatty alcohol as emulsifier.

13. The composition of claim 12, wherein the composition comprises a propellant and is dispensed as a mousse.

14. The composition of claim 12, wherein the composition comprises a propellant and is dispensed as an aerosol.

## Patentansprüche

1. Eine antimikrobielle chirurgische Reinigungszusammensetzung enthaltend:
a) 50 bis 95 Gew.-% eines C₂-C₄-Alkohols;
b) einen Konservierungsstoff ausgewählt aus der Gruppe bestehend aus phenolischen Verbindungen, Halogenverbindungen, quaternären Ammoniumverbindungen, Metallderivaten ausgewählt aus der Gruppe bestehend aus Silber-Borsilikat, Silber-Magnesium-Aluminium-Phosphat, Kupferusnat, Aminen, Alkanolaminen, Stickstoff enthaltende Verbindungen, Biguaniden, Aniliden, Organoschwefel-Verbindungen, Schwefel-Stickstoff-Verbindungen, und Mischungen davon;
c) ein Terpenoid ausgewählt aus der Gruppe bestehend aus Farnesol, Nerolidol, Bisabolol, Apriton, und Mischungen davon;
d) einen hautpflegenden Wirkstoff ausgewählt aus der Gruppe bestehend aus Weichmachern, Befeuchtungsmitteln, okkludierenden Wirkstoffen, und Mischungen davon;
e) ein Zellulose-haltiges Verdickungsmittel; und
f) einen Alkylenoxidether eines Fettalkohols als Emulgator,
wobei die Zusammensetzung eine Viskosität von 1800 bis 15000 Centipoise aufweist, bestimmt unter Verwendung eines Brookfield RVT Rotationsviskosimeters unter Verwendung einer Spindel #3 bei 10 UpM bei 25°C.

2. Die Zusammensetzung gemäß Anspruch 1, wobei der Alkohol Ethanol ist.

3. Die Zusammensetzung gemäß Anspruch 1, wobei
a) der Gehalt des Alkohols 50 bis 95 Gew.-% beträgt;
b) der Gehalt des Konservierungsstoffes 0,5 bis 3 Gew.-% beträgt;
c) der Gehalt des Terpenoides 0,005 bis 5 Gew.-% beträgt;
d) der Gehalt des hautpflegenden Wirkstoffes 0,01 bis 15 Gew.-% beträgt;
e) der Gehalt des Verdickungsmittels 0,01 bis 5 Gew.-% beträgt; und
f) der Gehalt des Emulgators 0,1 bis 8 Gew.-% beträgt.

4. Die Zusammensetzung gemäß Anspruch 1, wobei die Zusammensetzung weiterhin zusätzliche funktionelle Inhaltsstoffe ausgewählt aus der Gruppe bestehend aus Mitteln zur Verbesserung des Hautgefühls, Antioxidantien, Duftstoffen, Farbstoffen, und Mischungen davon enthält.

5. Die Zusammensetzung gemäß Anspruch 1, wobei die Zusammensetzung eine Viskosität von 2,000 bis 10,000 Centipoise aufweist.

6. Die Zusammensetzung gemäß Anspruch 1, wobei der Konservierungsstoff ausgewählt ist aus der Gruppe bestehend aus Polyaminopropylbiguanid, Benzethoniumchlorid, Benzalkoniumchlorid, und Mischungen davon.

7. Die Zusammensetzung gemäß Anspruch 1, wobei die Zusammensetzung eine Verringerung von *Serratia marcescens* von zumindest 2,5 log-Stufen innerhalb einer Einwirkdauer von 5 Minuten bewirkt.

8. Die Zusammensetzung gemäß Anspruch 1, wobei die Zusammensetzung eine Verringerung von *Serratia marcescens* von zumindest 3 log-Stufen innerhalb einer Einwirkdauer von 5 Minuten bewirkt.

9. Die Zusammensetzung gemäß Anspruch 1, wobei die Zusammensetzung bezüglich einer 5 log Ausgangspopulation physiologischer Flora eine Verringerung von zumindest 3,5 log-Stufen innerhalb einer Einwirkdauer von 1 Minute nach einer Benutzungsdauer von fünf Tagen bewirkt.

10. Die Zusammensetzung gemäß Anspruch 1, wobei die Zusammensetzung bezüglich einer 5 log Ausgangspopulation physiologischer Flora eine Verringerung von zumindest 4,0 log-Stufen innerhalb einer Einwirkdauer von 1 Minute nach einer Benutzungsdauer von fünf Tagen bewirkt.

11. Die Zusammensetzung gemäß Anspruch 1, wobei die Zusammensetzung im Wesentlichen frei von anionischen Wirkstoffen ist.

12. Eine antimikrobielle chirurgische Schaum-bildende
Reinigungszusammensetzung enthaltend:
a) 50 bis 95 Gew.-% eines C₂-C₄-Alkohols;
b) einen Konservierungsstoff ausgewählt aus der Gruppe bestehend aus phenolischen Verbindungen, Halogenverbindungen, quaternären Ammoniumverbindungen, Metallderivaten ausgewählt aus der Gruppe bestehend aus Silber-Borsilikat, Silber-Magnesium-Aluminium-Phosphat, Kupferusnat, Aminen, Alkanolaminen, Stickstoff enthaltende Verbindungen, Biguaniden, Aniliden, Organoschwefel-Verbindungen, Schwefel-Stickstoff-Verbindungen, und Mischungen davon;
c) ein Terpenoid ausgewählt aus der Gruppe bestehend aus Farnesol, Nerolidol, Bisabolol, Apriton, und Mischungen davon;
d) einen hautpflegenden Wirkstoff ausgewählt aus der Gruppe bestehend aus Weichmachern, Befeuchtungsmitteln, okkludierenden Wirkstoffen, und Mischungen davon;
e) ein Zellulose-haltiges Verdickungsmittel; und
f) ein Alkylenoxidether eines Fettalkohols als Emulgator.

13. Die Zusammensetzung gemäß Anspruch 12, wobei die Zusammensetzung ein Treibmittel enthält und als Mousse abgegeben wird.

14. Die Zusammensetzung gemäß Anspruch 12, wobei die Zusammensetzung ein Treibmittel enthält und als Aerosol abgegeben wird.

## Revendications

1. Composition antimicrobienne de nettoyage chirurgical comprenant :
a) de 50 à 95 % en poids d'un alcool en C₂ à C₄ ;
b) un conservateur choisi dans le groupe constitué par les dérivés phénoliques, les composés halogénés, les composés d'ammonium quaternaire, les dérivés de métaux choisis dans le groupe constitué par le borosilicate d'argent, le phosphate d'aluminium magnésium argent, l'usnate de cuivre, les amines, les alcanolamines, les composés contenant du nitro, les biguanides, les anilides, les composés organo-soufrés, les composés soufre-azote et leurs mélanges ;
c) un terpénoïde choisi dans le groupe constitué par le farnesol, le nérolidol, le bisabolol, l'apritone, et leurs mélanges ;
d) un conditionneur pour la peau choisi dans le groupe constitué par les émollients, les humectants, les agents occlusifs, et leurs mélanges ;
e) un épaississant cellulosique, et
f) un éther-oxyde d'alkylène d'alcool gras en tant qu'émulsifiant,
où la composition présente une viscosité de 1 800 à 15 000 centipoises telle que déterminée en utilisant un viscosimètre rotatif Brookfield RVT, ayant un fuseau n° 3 tournant à 10 tours/min à 25 °C.

2. Composition selon la revendication 1, dans laquelle l'alcool est de l'éthanol.

3. Composition selon la revendication 1, dans laquelle.
a) l'alcool est présent à hauteur de 50 à 95 % en poids ;
b) le conservateur est présent à hauteur de 0,5 à 3 % en poids ;
c) le terpénoïde est présent à hauteur de 0,005 à 5 % en poids ;
d) le conditionneur pour la peau est présent à hauteur de 0,01 à 15 % en poids ;
e) l'épaississant est présent à hauteur de 0,01 à 5 % en poids ;
f) l'émulsifiant est présent à hauteur de 0,1 à 8 % en poids.

4. Composition selon la revendication 1, dans laquelle la composition comprend en outre des ingrédients fonctionnels additionnels choisis dans le groupe constitué par les agents améliorant le toucher de la peau, les antioxydants, les parfums, les teintes, et leurs mélanges.

5. Composition selon la revendication 1, dans laquelle la composition présente une viscosité de 2 000 à 10 000 centipoises.

6. Composition selon la revendication 1, dans laquelle le conservateur est choisi dans le groupe constitué par le polyaminopropyl biguanide, le chlorure de benzéthonium, le chlorure de benzalkonium, et leurs mélanges.

7. Composition selon la revendication 1, dans laquelle la composition engendre au moins une réduction de 2,5 log de *Serratia marcescens* en l'espace de 5 minutes de contact.

8. Composition selon la revendication 1, dans laquelle la composition engendre au moins une réduction de 3 log de *Serratia marcescens* en l'espace de 5 minutes de contact.

9. Composition selon la revendication 1, dans laquelle la composition engendre au moins une réduction de 3,5 log d'au moins une population de ligne de base 5 log de flore résidente en l'espace d'une minute de contact après 5 jours d'utilisation.

10. Composition selon la revendication 1, dans laquelle la composition engendre au moins une réduction de 4 log d'au moins une population de ligne de base 5 log de la flore résidente en l'espace d'une minute de contact après 5 jours d'utilisation.

11. Composition selon la revendication 1, dans laquelle la composition est sensiblement exempte d'ingrédients anioniques.

12. Composition antimicrobienne de nettoyage chirurgical comprenant :
a) de 50 à 95 % en poids d'un alcool en C₂ à C₄ ;
b) un conservateur choisi dans le groupe constitué par les dérivés phénoliques, les composés halogénés, les composés d'ammonium quaternaire, les dérivés de métaux choisis dans le groupe constitué par le borosilicate d'argent, le phosphate d'aluminium magnésium argent, l'usnate de cuivre, les amines, les alcanolamines, les composés contenant du nitro, les biguanides, les anilides, les composés organo-soufrés, les composés soufre-azote et leurs mélanges ;
c) un terpénoïde choisi dans le groupe constitué par le farnesol, le nérolidol, le bisabolol, l'apritone, et leurs mélanges ;
d) un conditionneur pour la peau choisi dans le groupe constitué par les émollients, les humectants, les agents occlusifs, et leurs mélanges ;
e) un épaississant cellulosique, et
f) un éther-oxyde d'alkylène d'alcool gras en tant qu'émulsifiant.

13. Composition selon la revendication 12, dans laquelle la composition comprend un agent propulseur et est distribuée sous forme de mousse.

14. Composition selon la revendication 12, dans laquelle la composition comprend un agent propulseur et est distribuée sous forme d'aérosol.
